# EUROPÄISCHE PATENTSCHRIFT

(11) **EP 1 259 220 B1**
(45) Veröffentlichungstag und Bekanntmachung des Hinweises auf die Patenterteilung: **23.07.2008**
(21) Anmeldenummer: 01919314.3
(22) Anmeldetag: 20.02.2001
(51) Int. Cl.: A61Q 19/00, A61Q 19/10, C11D 17/00

(54) **WÄRMENDES HAUTREINIGUNGSGEL**
SKIN CLEANSING GEL HAVING A HEATING EFFECT
GEL NETTOYANT POUR LA PEAU A EFFET CHAUFFANT

(30) Priorität: 01.03.2000 DE 10009252
(43) Veröffentlichungstag der Anmeldung: 27.11.2002
(73) Patentinhaber: Henkel AG & Co. KGaA, 40589 Düsseldorf (DE)
(72) Erfinder: SCHOLZ, Wolfhard, 47829 Krefeld (DE); MEYER ZU SCHLOCHTERN-MARIC, Katrin, 49324 Melle (DE); WADLE, Armin, 40699 Erkrath (DE)
(86) Internationale Anmeldenummer: PCT/EP2001/001882
(87) Internationale Veröffentlichungsnummer: WO 2001/064176

(56) Entgegenhaltungen:
- EP-A- 0 518 721
- EP-A- 0 950 400
- WO-A-97/30148
- BE-A- 791 366
- DE-A- 3 141 746
- DE-A- 4 227 203
- DE-A- 19 624 870
- US-A- 5 747 004

## Beschreibung

Die Erfindung betrifft ein Reinigungsgel zur schonenden Reinigung der Haut, das bei der Anwendung mit Wasser vermischt wird und dabei Wärme freisetzt. Durch die freigesetzte Wärme kommt es zu einem sensorisch angenehmen Hauteindruck, einer Verbesserung des Reinigungseffektes, zu einer erhöhten Freisetzung der Duftstoffe und zu einer verbesserten Wirkung enthaltender hautkosmetischer Wirkstoffe.

Die Verwendung von wasserfreien Salzen mit negativer Lösungsenthalpie, die beim Lösen in Wasser Hydratationswärme freisetzen, zur Herstellung von kosmetischen Zubereitungen, die sich bei der Anwendung erwärmen, ist mehrfach beschrieben worden. Aus DE 2317140 C2 sind wässrige Haut- und Haarbehandlungsmittel bekannt, die kurz vor der Anwendung durch Hinzufügen von Calciumchlorid oder Magnesiumsulfat erwärmt werden. In DE 19624870 A1 und WO 97102802 A2 sind Zahnpflegemittel beschrieben, die bei Zutritt von Wasser oder Speichel beim Bürsten der Zähne Wärme freisetzen. In WO 93/08793 A1 ist ein Hautreinigungsmittel beschrieben, das z. B. als Gesichtsmaske angewendet werden kann und als wärmefreisetzende Komponente ein entwässertes Molekularsieb in einem wasserfreien Träger enthält. EP 950 400 A2 offenbart gelförmige Reinigungsmasken, die mittels aktiviertem Zeolith Hydratationswärme entwickeln und weitere, keine Hydratationswärme freisetzende Salze als Putzkörper enthalten. US 5747004 offenbart Zahnpflegemittel, die bei Zutritt von Wasser oder Speichel zu dem enthaltenen Natriumcarbonat Hydratationswärme freisetzen. BE 791366 offenbart wasserfreie gelförmige Oberflächenreiniger, die Natriumcarbonat enthalten.

Diese bekannten Zubereitungen sind aber zur Anwendung als Hautreinigungsmittel, z. B. in Form flüssiger oder pastenförmiger Zubereitungen vom Typ einer Flüssigseife oder einer Waschpaste weniger geeignet, da sie nicht genügend Schaum entwickeln und keine befriedigende Reinigung auch stärker verschmutzter Haut damit erzielt wird.

Es bestand daher ein Bedürfnis, ein Hautreinigungsmittel in Form einer gelförmigen, flüssigen bis hochviskosen Zubereitung zu entwickeln, das sich wie eine übliche Flüssigseife oder eine Handwaschpaste anwenden lässt und dabei einen voluminösen Schaum und gleichzeitig so viel Wärme entwickelt, dass ein sensorisch angenehmes Hautgefühl und eine porentiefe Reinigung der Haut erreicht wird.

Die gestellte Aufgabe wurde gelöst durch ein Reinigungsgel, das beim Vermischen mit Wasser Hydrationswärme freisetzt, enthaltend einen wasserfreien flüssigen Träger und darin dispergierte Pulverkomponenten und **gekennzeichnet durch** einen Gehalt von
(A) wenigstens 40 Gew.-% wassermischbaren Hydroxylverbindungen, ausgewählt aus Glycolen, Glycolethern und Polyolen mit jeweils 2 bis 6 C-Atomen, Polyalkylenglycolen mit Molekulargewichten bis 1000 D und Gemischen davon,
(B) wenigstens 5 Gew.-% anionischen, zwitterionischen, amphoteren oder nichtionischen Tensiden,
(C) wenigstens 5 Gew.-% dispergierten, teilchenförmigen, wasserlöslichen, ganz oder teilweise dehydratisierten Salzen mit negativer Lösungsenthalpie (in Wasser), ausgewählt aus den Sulfaten von Alkalimetallen, Calciumchlorid, Magnesiumchlorid, Magnesiumsulfat und Aluminiumsulfat oder Gemischen davon,
(D) wenigstens 0,1 Gew.-% eines im Träger gelösten wasserlöslichen Verdickungsmittels oder wenigstens 1 Gew.-% eines im Träger dispergierten teilchenförmigen Verdickungsmittels oder beiden, sowie
(E) wenigstens 3 Gew.-% eines teilchenförmigen, inerten Adsorptionsmittels, ausgewählt aus Talkum, Cellulose, modifizierter Stärke und Polyamidpulver.

Als wasserfrei wird dabei eine Trägerflüssigkeit angesehen, die so wenig Wasser enthält, dass keine nennenswerte Hydratisierung der darin dispergierten hydratisierenden Salze erfolgt.

Wassermischbare Hydroxylverbindungen im Sinne der Erfindung sind vor allem Glycole, Glycolether und Polyole mit jeweils 2-6 C-Atomen. Geeignete Glycole sind daher Ethylenglycol, Propandiole und Butandiole. Geeignete Glycolether sind z. B. Ethylglycol, Ethyldiglycol, Diethylenglycol, Triethylenglycol und Dipropylenglycol. Geeignete Polyole sind z. B. Glycerin, Erythrit, Pentaerythrit, Trimethylolpropan, Diglycerin und Sorbit. Geeignete Polyalkylenglycole sind z. B. die flüssigen Polyethylenglycole, die Polypropylenglycole und die Anlagerungsprodukte von Ethylenoxid an Propylenglycol oder an Polypropylenglycole, jeweils mit Molekulargewichten bis ca. 1000 D. Einwertige Alkohole wie z. B. Ethanol oder Isopropanol können ebenfalls in begrenzten Mengen bis ca. 5 Gew.-% enthalten sein.

Bevorzugt sind solche wassermischbaren Hydroxylverbindungen in einer Menge von 50 - 70 Gew.-% des Reinigungsgels enthalten. Durch die Art und Menge der wassermischbaren Hydroxylverbindungen läßt sich die Konsistenz sowie das Löse- und Dispergierverhalten des Trägers beeinflussen. Darüber hinaus haben einige dieser Hydroxylverbindungen ebenfalls eine negative Mischungsenthalpie mit Wasser, d. h. beim Mischen mit Wasser wird Wärme freigesetzt. Aus diesem Grund ist es bevorzugt, Mischungen von zwei oder mehr unterschiedlichen Hydroxylverbindungen einzusetzen um ein optimales Anwendungsprofil zu erhalten. Gemische von Propylenglycol-1,2, Butylenglycol-1,3, Polyethylenglycol und Ethoxy-diglycol sind besonders bevorzugt geeignete Träger.

Als anionische Tenside eignen sich alle oberflächenaktiven Stoffe, deren Oberflächenaktivität durch ein Anion bedingt ist, das sich durch eine bevorzugt lineare Alkyl- oder Acylgruppe mit 10-18 C-Atomen auszeichnet, die mit einer Sulfat-, Sulfonat-, Phosphat- oder Carboxylat-Gruppe verknüpft ist. Bevorzugt eignen sich schäumende anionische Tenside wie z. B. die Alkylsulfate, die Alkansulfonate, die Alpha-Olefinsulfonate, die Acylisethionate, die Acyltauride, die Acylsarkoside, die Sulfobernsteinsäuremonoalkylester-Salze, die Alkylpolyglycolethercarboxylate, in Form ihrer Alkali-, Magnesium-, Ammonium- oder Alkanolammoniumsalze. Bevorzugt werden solche anionischen Tenside eingesetzt, die in wasserfreier, feinteiliger Form zugänglich sind. Dies sind in der Regel die Natriumsalze der genannten Aniontenside. Als zwitterionische Tenside eignen sich vor allem die Betain-Tenside, z. B. das C₁₂-C₁₈-Alkyl-dimethyl-acetobetain, das Kokoamidopropyl-dimethyl-acetobetain, Imidazoliniumbetaine und Sulfobetaine mit einer bevorzugt linearen Alkyl- oder Acylgruppe mit 10-18 C-Atomen. Bevorzugt geeignet sind vor allem solche Betain-Tenside, die in wasserfreier, feinteiliger Form verfügbar sind. Ein besonders geeignetes Produkt ist z. B. das als Tego Betain CKD im Handel erhältliche Kokoamidopropylbetain (N,N-Dimethyl-N-(lauroylamidopropyl)-ammoniumacetobetain).

Als ampholytische Tenside werden solche oberflächenaktiven Stoffe verstanden, die neben einer bevorzugten linearen Alkyl- oder Acylgruppe mit 8-18 C-Atomen eine protonierbare Aminogruppe und eine Carboxylgruppe enthalten und zur Ausbildung innerer Salze befähigt sind. Geeignete ampholytische Tenside sind z. B. N-(C₁₂-C₁₈)-alkyl-N-methyl-glycin, N-(C₁₂-C₁₈)-acylaminopropyl-N-methyl-glycin, N-(C₁₂-C₁₈)-acyl-aminoethyl-N-methyl-glycin, N-(C₁₂-C₁₈)-acylaminopropyl-N-hydroxyethyl-glycin, 2-(C₁₂-C₁₈)-alkyl-carboxymethyl-3-hydroxylethyl-imidazolin und 2-N-(C₈-C₁₈)-alkylaminoethancarbonsäure.

Als nichtionische Tenside werden solche Tenside verstanden, die eine lipophile, bevorzugt lineare Alkyl- oder Acylgruppe mit 8-22 C-Atomen und als hydrophile Gruppe einen Glucosid- oder Polyglucosidrest, einen Glycerin- oder Polyglycerinrest, einen Sorbitanrest oder einen Polyglycoletherrest oder mehrere dieser Reste enthalten. Geeignete nichtionogene Tenside sind vor allem solche, die in wasserfreier Form zur Verfügung stehen, z. B. die Anlagerungsprodukte von Ethylenoxid an Fettalkohole, an Fettsäuren, an Fettsäuremono- oder diglyceride, an Fettsäurealkanolamide, an Sorbitanfettsäureester, an Methylglucosidfettsäureester oder an Alkylglucoside. Eine weitere besonders geeignete Gruppe nichtionischer Tenside sind die Siliconcopolyole, die z. B. unter der Handelsbezeichnung Dow Coming Surfactant (Dow Corning) oder Abil (Goldschmidt) im Handel sind.

In einer bevorzugten Ausführung enthält das erfindungsgemäße Reinigungsgel als Tensid ein im Träger dispergiertes, anionisches oder zwitterionisches Tensid oder ein Gemisch solcher Tenside in einer Menge von 10-30 Gew.-%.

Als hydratisierende Salze mit negativer Lösungsenthalpie sind solche wasserlöslichen Salze zu verstehen, die sich in Wasser unter Wärmeentwicklung lösen. Dies ist in der Regel dann der Fall, wenn bei der Auflösung Hydrate gebildet werden und die Bildungswärme dieser Hydrate größer ist als die zur Überwindung der Gitterenergie verbrauchte Wärme. In der Regel handelt es sich dabei um ganz oder teilweise dehydratisierte Salze, die in Wasser Hydrate bilden. Solche Salze sind z. B. Sulfate von Alkalimetallen, z.B. des Natriums. Weitere geeignete Salze sind Calciumchlorid, Magnesiumchlorid, Magnesiumsulfat und Aluminiumsulfat.

In einer bevorzugten Ausführung der Erfindung ist als hydratisierendes Salz Natrium-, Magnesium- oder Aluminiumsulfat oder ein Gemisch davon in einer Menge von 5 - 20 Gew.-% enthalten. Dabei ist darauf zu achten, dass Salze mit hoher Lösungswärme nicht in zu hoher Dosierung eingesetzt werden, damit es bei der Anwendung nicht zu einer unangenehmen Hitzeentwicklung auf der Haut kommt. Daher sollte z. B. MgSO₄ (wasserfrei) in Mengen von nicht mehr als 15 Gew.-% eingesetzt werden.

Die gelartige Konsistenz des erfindungsgemäßen Reinigungsmittels lässt sich durch den Einsatz geeigneter Verdickungsmittel steuern. Bevorzugt sollte die Viskosität des Produktes höher als 2 Pa·s (gemessen mit einem Brookfield Rotationsviskosimeter, Typ RTV-Helipath, Spindel D, bei 20 UpM, 20°C) liegen. Für die Anwendung des erfindungsgemäßen Reinigungsgels aus Tuben oder flexiblen Kunststoff-Spendefläschchen ist eine Viskosität von mehr als 5 Pa·s, insbesondere von 10-50 Pa·s, bevorzugt.

Die im Träger gelösten Verdickungsmittel sind üblicherweise organische Hydrocolloide, also natürliche oder synthetische Polymere, die in Wasser quellbar oder löslich sind und die sich in der im wesentlichen aus den vorgenannten Hydroxyverbindungen bestehenden Trägerflüssigkeit lösen lassen. Geeignete gelöste Verdickungsmittel sind z. B. nichtionische Polysaccharidderivate wie z. B. Hydroxypropylcellulose, Hydroxypropylstärke, Hydroxypropylguar oder synthetische Polymere wie Polyvinylpyrrolidon, Polyvinylalkohol oder Polyacrylamid. In einer bevorzugten Ausführung ist in dem erfindungsgemäßen Reinigungsgel als im Träger gelöstes Verdickungsmittel ein nichtionogenes Polysaccharidderivat in einer Menge von 0,1 bis 1 Gew.-% enthalten.

Als im Träger dispergierte, teilchenförmige Verdickungsmittel werden z. B. Schichtsilikate wie z. B. Tone (Kaoline, Montmorillonite) oder amorphe Kieselsäuren wie z. B. pyrogene Kieselsäuren (Aerosil) oder besonders feinteilige Gelkieselsäuren verwendet. In einer bevorzugten Ausführung enthält das erfindungsgemäße Reinigungsgel als im Träger dispergiertes Verdickungsmittel eine amorphe Kieselsäure in einer Menge von 1-5 Gew.-%.

Darüber hinaus sind in dem Reinigungsgel auch andere, weniger verdickende, aber adsorptiv wirksame, pulverförmige Stoffe enthalten, die sich durch eine große Oberfläche, also durch eine relativ niedrige Teilchengröße auszeichnen. Solche Stoffe können Talkum, Cellulose, modifizierte Stärke oder Polymerpulver wie z. B. Polyamidpulver sein. Das erfindungsgemäße Reinigungsgel enthält wenigstens 3 Gew.-% eines teilchenförmigen, inerten Adsorptionsmittels. Ein besonders bevorzugtes teilchenförmiges Adsorptionsmittel ist Talkum, das in Mengen von 3 - 30 Gew.-% in dem Reinigungsgel enthalten sein kann.

In einer besonders bevorzugten Ausführung enthält das erfindungsgemäße Reinigungsgel

| | |
|---|---|
| 45 - 55 Gew.-% | eines Gemisches aus 1,2-Propylenglycol oder 1,3-Butylenglycol, Polyethylenglycol und Ethoxydiglycol |
| 10 - 30 Gew.-% | dispergierte anionische oder zwitterionische Tenside |
| 0,5 - 5 Gew.-% | gelöste nichtionische Tenside |
| 5 - 20 Gew.-% | Natriumsulfat, Magnesiumsulfat oder Aluminiumsulfat oder eines Gemisches davon |
| 0,1 - 0,5 Gew.-% | gelöste, nichtionische Celluloseether |
| 1 - 5 Gew.-% | amorphe Kieselsäure und |
| 3 - 20 Gew.-% | Talkum. |

Zusätzlich zu den genannten Komponenten können in dem erfindungsgemäßen Reinigungsmittel weitere in Körperreinigungsmitteln übliche Hilfsmittel und Zusätze enthalten sein. Solche Zusätze dienen der Verbesserung der Hautverträglichkeit und der kosmetischen Sensorik ganz allgemein. Geeignete Stoffe sind z. B. Feuchthaltemittel wie z. B. Pyrrolidoncarbonsäure, keratolytische und hautweichmachende Komponenten wie z. B. Harnstoff, Allantoin, rückfettende Komponenten wie z. B. emulgierte Lipidkomponenten und Silikone, dispergierte Fette und Wachse, insbesondere solche, die in mikroemulgierter oder nanopartikulärer Form vorliegen, Vitamine wie Tocopherol, Retinol oder Ascorbinsäure, Panthenol oder Biotin, wasserlösliche Proteinderivate, Duftstoffe, Farbstoffe und Perlglanzpigmente.

Weitere Hilfsmittel, die vorwiegend der Lagerstabilität dienen, sind z. B. Konservierungsstoffe, Komplexbildner, pH-Regulatoren und Puffersubstanzen.

Die Herstellung der erfindungsgemäßen Reinigungsgele erfolgt durch einfaches Vermischen der Komponenten unter Erwärmen. Dabei legt man die flüssigen Trägerkomponenten, also die Hydroxylverbindungen vor und löst oder dispergiert darin die Verdikkungsmittel, dann fügt man nacheinander die löslichen, nichtionischen Tenside, die pulverförmigen, zu dispergierenden Tenside, die Adsorbentien und zuletzt die hydratisierenden Salze zu. Es empfiehlt sich, dabei in einer geschlossenen Anlage unter leichtem Unterdruck zu arbeiten, damit im Produkt keine Luft eingeschlossen wird und nicht zu viel Luftfeuchtigkeit zutreten kann.

Die folgenden Beispiele sollen den Gegenstand der Erfindung näher erläutern:

### Beispiele

Es wurden folgende Zusammensetzungen hergestellt (Anteil in Gew.-%):

| | Vergleichs-beispiel | | | | |
|---|---|---|---|---|---|
| | 1 | 2 | 3 | 4 | 5 |
| 1,2-Propylenglycol | - | 25 | 30 | 25 | 25 |
| 1,3-Butylenglycol | 35 | - | - | 5 | - |
| Polyethylenglycol 400 | 10 | 20 | 20 | 15 | 25 |
| Ethoxydiglycol | 8,0 | 10 | 10 | 5,0 | 10 |
| Glycerin | 3,0 | 2,0 | 2,0 | 2,0 | - |
| Dow Corning^{®} 193 | 1,0 | 1,0 | - | 1,0 | 1,0 |
| Cetiol^{®} HE | - | 1,0 | 1,5 | - | 2,0 |
| Elfan^{®} AT 84 | 5,0 | - | 10 | 20 | - |
| Tego Betain^{®} CKD | - | 15 | 10 | - | 15 |
| Sulfosuccinat 128 P | 15,0 | - | 3,0 | - | 5,0 |
| Parfümöl | 0,3 | 0,3 | 0,3 | 0,3 | 0,3 |
| Klucel^{®} M | - | 0,2 | 0,2 | 0,3 | 0,3 |
| Steasilk^{®} 5-GG-HT | 3,7 | 11,3 | - | 16,4 | 4,4 |
| Neosil^{®} CT 11 | - | 2,5 | - | - | 2,0 |
| Aerosil^{®} 200 | 4,0 | - | 3,0 | 2,0 | - |
| Na₂SO₄ | 15 | - | - | - | - |
| Mg SO₄ | - | 12 | 10,0 | 8,0 | - |
| (Al)₂ (SO₄)₃ | - | - | - | - | 10 |
| Viskosität (Pa·s) | 38 | 4 | 11 | 29 | 9 |
| (Brookfield, Type RTV, Spindel D, 20 UpM, 20°C) | | | | | |

Es wurden folgende Handelsprodukte verwendet:

| | |
|---|---|
| Cetiol^{®} HE (Cognis Deutschland): | Glycerin +7,3 EO-Kokosfettsäureester |
| Elfan^{®} AT 84 (Akzo Nobel): | Kokosacylisethionat, Na-Salz (Pulver 84 Gew.-% AS) |
| Tego Betain^{®} CKD (Goldschmidt): | Kokosacylamidopropyl-Betain (Pulver 82 Gew.-% AS) |
| Sulfosuccinat 128 P (Cognis Deutschland): | Sulfobernsteinsäure-monofettalkyl (C₁₂₋₁₈)-ester, Di-Na-Salz (Pulver, 90 % AS) |
| Klucel^{®} M (Hercules): | Hydroxypropylcellulose (Pulver) |
| Steasilk^{®} 5-GG-HT (Luzenac, NV): | Talkum (Pulver) |
| Neosil^{®} CT11: (Crosfield) | Kieselsäure, amorph |
| Aerosil^{®} 200 (Degussa): | Kieselsäure, pyrogen, amorph |

## Patentansprüche

1. Reinigungsgel, das beim Vermischen mit Wasser Hydrationswärme erzeugt, enthaltend einen wasserfreien, flüssigen Träger, darin dispergierte Pulverkomponenten sowie weiterhin
(A) wenigstens 40 Gew.% wassermischbaren Hydroxylverbindungen, ausgewählt aus Glycolen, Glycolethern, Polyolen mit jeweils 2 - 6 C-Atomen, Polyalkylenglycolen mit mittleren Molekulargewichten bis 1000 D und Gemischen davon,
(B) wenigstens 5 Gew.-% anionischen, zwitterionischen, ampholytischen oder nichtionischen Tensiden,
(C) wenigstens 5 Gew.-% dispergierten, teilchenförmigen, wasserlöslichen, ganz oder teilweise dehydratisierten Salzen mit negativer Lösungsenthalpie (in Wasser), ausgewählt aus den Sulfaten von Alkalimetallen, Calciumchlorid, Magnesiumchlorid, Magnesiumsulfat und Aluminiumsulfat oder Gemischen davon, sowie
(D) wenigstens 0,1 Gew.-% eines im Träger gelösten, wasserlöslichen Verdickungsmittels oder wenigstens 1 Gew.-% eines im Träger dispergierten teilchenförmigen Verdickungsmittels,
(E) wenigstens 3 Gew.-% eines teilchenförmigen, inerten Adsorptionsmittels, ausgewählt aus Talkum, Cellulose, modifizierter Stärke und Polymerpulvern, wie z. B. Polyamidpulver.

2. Reinigungsgel gemäß Anspruch 1, **dadurch gekennzeichnet, dass** die wassermischbaren Hydroxylverbindungen in einer Menge von 50 - 70 Gew.-% des Reinigungsgels enthalten sind.

3. Reinigungsgel gemäß einem der Ansprüche 1 oder 2, **dadurch gekennzeichnet, dass** als Tensid ein im Träger dispergiertes anionisches oder zwitterionisches Tensid oder ein Gemisch solcher Tenside in einer Menge von 10 - 30 Gew.-% enthalten ist.

4. Reinigungsgel gemäß einem der Ansprüche 1 - 3, **dadurch gekennzeichnet, dass** als hydratisierendes Salz (C) Natrium-, Magnesium- oder Aluminiumsulfat oder ein Gemisch davon in einer Menge von 5 - 20 Gew.-% enthalten ist, wobei nicht mehr als 15 Gew.-% Magnesiumsulfat enthalten ist.

5. Reinigungsgel gemäß einem der Ansprüche 1 - 4, **dadurch gekennzeichnet, dass** das im Träger gelöste Verdickungsmittel ausgewählt ist aus organischen Hydrocolloiden.

6. Reinigungsgel gemäß Anspruch 5, **dadurch gekennzeichnet, dass** das im Träger gelöste Verdickungsmittel ausgewählt ist aus nichtionischen Polysaccharidderivaten, wie z. B. Hydroxypropylcellulose, Hydroxypropylstärke, Hydroxypropylguar, oder synthetischen Polymeren wie Polyvinylpyrrolidon, Polyvinylalkohol oder Polyacrylamid.

7. Reinigungsgel gemäß einem der Ansprüche 1 - 6, **dadurch gekennzeichnet, dass** als im Träger gelöstes Verdickungsmittel ein nichtionogenes Polysaccharidderivat in einer Menge von 0,1 bis 1 Gew.-% enthalten ist.

8. Reinigungsgel gemäß einem der Ansprüche 1 - 7, **dadurch gekennzeichnet, dass** als im Träger dispergiertes Verdickungsmittel eine amorphe Kieselsäure in einer Menge von 1 - 5 Gew.-% enthalten ist.

9. Reinigungsgel gemäß einem der Ansprüche 1 - 8, **dadurch gekennzeichnet, dass** das nichtionische Tensid ausgewählt ist aus Siliconcopolyolen.

10. Reinigungsgel gemäß einem der Ansprüche 1 - 9, **dadurch gekennzeichnet, dass** es eine Viskosität von mehr als 2 Pa·s, bevorzugt mehr als 5 Pa·s, insbesondere von 10 - 50 Pa·s, (gemessen mit einem Brookfield Rotationsviskosimeter, Typ RTV-Helipath, Spindel D, bei 20 UpM und 20°C) aufweist.

11. Reinigungsgel gemäß einem der Ansprüche 1 - 10, **dadurch gekennzeichnet, dass** Talkum in einer Menge von 3 - 30 Gew.-% enthalten ist.

12. Reinigungsgel gemäß Anspruch 1, **gekennzeichnet durch** einen Gehalt von
| | | |
|---|---|---|
| 45 - 55 | Gew. % | eines Gemisches aus Propylenglycol oder Butylenglycol, Polyethylenglycol und Ethoxydiglycol, |
| 10-30 | Gew. % | eines dispergierten anionischen oder zwitterionischen Tensids, |
| 0,5 - 5 | Gew. % | eines gelösten nichtionischen Tensids |
| 5 - 20 | Gew. % | Magnesiumsulfat oder Natriumsulfat, |
| 0,1 - 0,5 | Gew. % | eines gelösten nichtionischen Celluloseesters, |
| 1 - 5 | Gew. % | einer amorphen Kielselsäure, |
| 3 - 20 | Gew. % | Talkum. |

13. Reinigungsgel gemäß Anspruch 1, **gekennzeichnet durch** einen Gehalt von
| | | |
|---|---|---|
| 45 - 55 | Gew. % | eines Gemisches aus 1,2-Propylenglycol oder 1,3-Butylenglycol, Polyethylenglycol und Ethoxydiglycol, |
| 10 - 30 | Gew. % | dispergierte anionische oder zwitterionische Tenside, |
| 0,5 - 5 | Gew. % | gelöste nichtionische Tenside, |
| 5 - 20 | Gew. % | Natriumsulfat, Magnesiumsulfat oder Aluminiumsulfat oder eines Gemisches davon, |
| 0,1 - 0,5 | Gew. % | gelöste nichtionische Celluloseether, |
| 1 - 5 | Gew. % | amorphe Kielselsäure, |
| 3 - 20 | Gew. % | Talkum. |

## Claims

1. Cleansing gel which, upon mixing with water, generates heat of hydration, comprising a water-free, liquid carrier, powder components dispersed therein, and also
(A) at least 40% by weight of water-miscible hydroxyl compounds selected from glycols, glycol ethers, polyols having in each case 2-6 carbon atoms, polyalkylene glycols with average molecular weights up to 1000 D and mixtures thereof,
(B) at least 5% by weight of anionic, zwitterionic, ampholytic or nonionic surfactants,
(C) at least 5% by weight of dispersed, particulate, water-soluble, completely or partially dehydrated salts with negative enthalpy of solution (in water), selected from the sulphates of alkali metals, calcium chloride, magnesium chloride, magnesium sulphate and aluminium sulphate or mixtures thereof, and
(D) at least 0.1% by weight of a water-soluble thickener dissolved in the carrier, or at least 1% by weight of a particulate thickener dispersed in the carrier,
(E) at least 3% by weight of a particulate, inert adsorbent selected from talc, cellulose, modified starch and polymer powders, such as, for example, polyamide powder.

2. Cleansing gel according to Claim 1, **characterized in that** the water-miscible hydroxyl compounds are present in an amount of 50-70% by weight of the cleansing gel.

3. Cleansing gel according to one of Claims 1 or 2, **characterized in that** the surfactant present is an anionic or zwitterionic surfactant dispersed in the carrier or a mixture of such surfactants in an amount of 10-30% by weight.

4. Cleansing gel according to one of Claims 1-3, **characterized in that** the hydrating salt (C) present is sodium sulphate, magnesium sulphate or aluminium sulphate or a mixture thereof in an amount of 5-20% by weight, where not more than 15% by weight of magnesium sulphate is present.

5. Cleansing gel according to one of Claims 1-4, **characterized in that** the thickener dissolved in the carrier is selected from organic hydrocolloids.

6. Cleansing gel according to Claim 5, **characterized in that** the thickener dissolved in the carrier is selected from nonionic polysaccharide derivatives, such as, for example, hydroxypropyl cellulose, hydroxypropyl starch, hydroxypropyl guar, or synthetic polymers, such as polyvinylpyrrolidone, polyvinyl alcohol or polyacrylamide.

7. Cleansing gel according to one of Claims 1-6, **characterized in that** the thickener dissolved in the carrier present is a nonionogenic polysaccharide derivative in an amount of from 0.1 to 1% by weight.

8. Cleansing gel according to one of Claims 1-7, **characterized in that** the thickener dispersed in the carrier present is an amorphous silica in an amount of 1-5% by weight.

9. Cleansing gel according to one of Claims 1-8, **characterized in that** the nonionic surfactant is selected from silicone copolyols.

10. Cleansing gel according to one of Claims 1-9, **characterized in that** it has a viscosity of more than 2 Pa·s, preferably more than 5 Pa·s, in particular of 10-50 Pa·s (measured using a Brookfield rotary viscometer, model RTV-Helipath, spindle D, at 20 rpm and 20°C).

11. Cleansing gel according to one of Claims 1-10, **characterized in that** talc is present in an amount of 3-30% by weight.

12. Cleansing gel according to Claim 1, **characterized by** a content of
| | |
|---|---|
| 45-55% by weight | of a mixture of propylene glycol or butylene glycol, polyethylene glycol and ethoxydiglycol, |
| 10-30% by weight | of a dispersed anionic or zwitterionic surfactant, |
| 0.5-5% by weight | of a dissolved nonionic surfactant |
| 5-20% by weight | of magnesium sulphate or sodium sulphate, |
| 0.1-0.5% by weight | of a dissolved nonionic cellulose ester, |
| 1-5% by weight | of an amorphous silica, |
| 3-20% by weight | of talc. |

13. Cleansing gel according to Claim 1, **characterized by** a content of
| | |
|---|---|
| 45-55% by weight | of a mixture of 1,2-propylene glycol or 1,3-butylene glycol, polyethylene glycol and ethoxydiglycol, |
| 10-30% by weight | of dispersed anionic or zwitterionic surfactants, |
| 0.5-5% by weight | of dissolved nonionic surfactants, |
| 5-20% by weight | of sodium sulphate, magnesium sulphate or aluminium sulphate or a mixture thereof, |
| 0.1-0.5% by weight | of dissolved nonionic cellulose ethers, |
| 1-5% by weight | of amorphous silica, |
| 3-20% by weight | of talc. |

## Revendications

1. Gel nettoyant qui, lorsqu'on le mélange à l'eau, produit une chaleur d'hydratation, contenant un support liquide anhydre, des composants pulvérulents qui y sont dispersés ainsi qu'en outre
(A) au moins 40 % en poids de composés hydroxyle miscibles à l'eau, choisis parmi les glycols, les éthers de glycols, les polyols comportant à chaque fois de 2 à 6 atomes de carbone, les polyalkylèneglycols ayant des poids moléculaires moyens allant jusqu'à 1000 D et leurs mélanges,
(B) au moins 5 % en poids d'agents tensioactifs anioniques, zwittérioniques, ampholytiques ou non ioniques,
(C) au moins 5 % en poids de sels dispersés, particulaires, solubles dans l'eau, entièrement ou partiellement déshydratés avec une enthalpie de dissolution négative (dans l'eau), choisis parmi les sulfates de métaux alcalins, le chlorure de calcium, le chlorure de magnésium, le sulfate de magnésium et le sulfate d'aluminium ou leurs mélanges, ainsi que
(D) au moins 0,1 % en poids d'un épaississant soluble dans l'eau, dissous dans le support, ou au moins 1 % en poids d'un épaississant particulaire dispersé dans le support,
(E) au moins 3 % en poids d'un adsorbant inerte particulaire choisi parmi le talc, la cellulose, l'amidon modifié et les poudres polymères, comme par exemple la poudre de polyamide.

2. Gel nettoyant selon la revendication 1, **caractérisé en ce qu'**il contient les composés hydroxyle miscibles à l'eau en une quantité de 50 à 70 % en poids du gel nettoyant.

3. Gel nettoyant selon l'une des revendications 1 ou 2, **caractérisé en ce qu'**il contient comme agent tensioactif un agent tensioactif anionique ou zwittérionique dispersé dans le support ou un mélange de tels agents tensioactifs en une quantité de 10 à 30 % en poids.

4. Gel nettoyant selon l'une des revendications 1 à 3, **caractérisé en ce qu'**il contient comme sel hydratant (C) un sulfate de sodium, de magnésium ou d'aluminium, ou un de leurs mélanges, en une quantité de 5 à 20 % en poids, où le sulfate de magnésium est contenu en une proportion ne dépassant pas 15 % en poids.

5. Gel nettoyant selon l'une des revendications 1 à 4, **caractérisé en ce que** l'épaississant dissous dans le support est choisi parmi les hydrocolloïdes organiques.

6. Gel nettoyant selon la revendication 5, **caractérisé en ce que** l'épaississant dissous dans le support est choisi parmi les dérivés de polysaccharides non ioniques, comme par exemple l'hydroxypropylcellulose, l'hydroxypropylamidon, l'hydroxypropylguar ou les polymères synthétiques comme la polyvinylpyrrolidone, l'alcool polyvinylique ou le polyacrylamide.

7. Gel nettoyant selon l'une des revendications 1 à 6, **caractérisé en ce qu'**il contient comme épaississant dissous dans le support un dérivé de polysaccharide non ionogène en une quantité de 0,1 à 1 % en poids.

8. Gel nettoyant selon l'une des revendications 1 à 7, **caractérisé en ce qu'**il contient comme épaississant dissous dans le support un acide silicique amorphe en une quantité de 1 à 5 % en poids.

9. Gel nettoyant selon l'une des revendications 1 à 8, **caractérisé en ce que** l'agent tensioactif non ionique est choisi parmi les copolyols de silicone.

10. Gel nettoyant selon l'une des revendications 1 à 9, **caractérisé en ce qu'**il présente une viscosité supérieure à 2 Pa.s, de préférence supérieure à 5 Pa.s, en particulier de 10 à 50 Pa.s, (mesurée avec un viscosimètre à rotation Brookfield, type RTV-Helipath, arbre D, à 20 tpm et à 20 °C).

11. Gel nettoyant selon l'une des revendications 1 à 10, **caractérisé en ce qu'**il contient du talc en une quantité de 3 à 30 % en poids.

12. Gel nettoyant selon la revendication 1, **caractérisé en ce qu'**il contient
45 à 55 % en poids d'un mélange de propylèneglycol ou de butylèneglycol, de polyéthylèneglycol et d'éthoxydiglycol,
10 à 30 % en poids d'un agent tensioactif anionique ou zwittérionique dispersé,
0,5 à 5 % en poids d'un agent tensioactif non ionique dissous,
5 à 20 % en poids de sulfate de magnésium ou de sulfate de sodium,
0,1 à 0,5 % en poids d'un ester de cellulose non ionique dissous,
1 à 5 % en poids d'un acide silicique amorphe,
3 à 20 % en poids de talc.

13. Gel nettoyant selon la revendication 1, **caractérisé en ce qu'**il contient
45 à 55 % en poids d'un mélange de 1,2-propylèneglycol ou de 1,3-butylèneglycol, de polyéthylèneglycol et d'éthoxydiglycol,
10 à 30 % en poids d'agents tensioactifs anioniques ou zwittérioniques dispersés,
0,5 à 5 % en poids d'agents tensioactifs non ioniques dissous,
5 à 20 % en poids de sulfate de sodium, de sulfate de magnésium ou de sulfate d'aluminium ou d'un de leurs mélanges,
0,1 à 0,5 % en poids d'éthers de cellulose non ioniques dissous,
1 à 5 % en poids d'acide silicique amorphe,
3 à 20 % en poids de talc.
